# EUROPÄISCHE PATENTSCHRIFT

(11) **EP 2 384 138 B1**
(45) Veröffentlichungstag und Bekanntmachung des Hinweises auf die Patenterteilung: **26.09.2018**
(21) Anmeldenummer: 09765044.4
(22) Anmeldetag: 08.12.2009
(51) Int. Cl.: A61B 5/021, A61M 1/36

(54) **VORRICHTUNG ZUM ERMITTELN UND/ODER ÜBERWACHEN EINER FISTELDURCHGÄNGIGKEIT BASIEREND AUF EINER AMPLITUDE DES HERZDRUCKSIGNALS WÄHREND EINER BLUTBEHANDLUNG**
DEVICE FOR DETERMINING AND/OR MONITORING FISTULA PATENCY ON THE BASIS OF AN AMPLITUDE OF THE HEART PRESSURE SIGNAL DURING A BLOOD TREATMENT
DISPOSITIF PERMETTANT DE DÉTERMINER ET/OU SURVEILLER LA PERMÉABILITÉ D'UNE FISTULE À PARTIR DE L'AMPLITUDE D'UN SIGNAL DE LA PRESSION CARDIAQUE LORS D'UN TRAITMENT DU SANG

(30) Priorität: 09.12.2008 DE 102008061122
(43) Veröffentlichungstag der Anmeldung: 09.11.2011
(73) Patentinhaber: Fresenius Medical Care Deutschland GmbH, 61352 Bad Homburg (DE)
(72) Erfinder: GRÖBER, Tobias, 63150 Heusenstamm (DE); MOISSL, Ulrich, 61184 Karben (DE); WABEL, Peter, 64287 Darmstadt (DE)
(74) Vertreter: Bobbert & Partner Patentanwälte PartmbB
(86) Internationale Anmeldenummer: PCT/EP2009/008765
(87) Internationale Veröffentlichungsnummer: WO 2010/066405

(56) Entgegenhaltungen:
- EP-B1- 0 957 956
- DE-C1- 19 901 078
- US-A1- 2006 074 369
- ODA MASAMI ET AL: "New blood volume monitoring method for hemodialysis: A-V pressure gradient measurement by synchronized one-point reading." ARTIFICIAL ORGANS JUL 2004, Bd. 28, Nr. 7, Juli 2004 (2004-07), Seiten 683-689, XP002573166 ISSN: 0160-564X
- P. WABEL ET AL: "Ansätze zur Identifikation von Patientenparametern während der Hämodialysetherapie" AT - AUTOMATISIERUNGSTECHNIK, Bd. 50, 2002, Seiten 220-227, XP009130863
- VASCULAR ACCESS 2006 WORK GROUP: "Clinical practice guidelines for vascular access." AMERICAN JOURNAL OF KIDNEY DISEASES, Bd. 48 Suppl 1, Juli 2006 (2006-07), Seiten S176-S247, XP002583666 ISSN: 1523-6838
- ULRICH MOISSL: "Kardiovaskuläre Überwachung bei der Hämodialysetherapie" FORTSCHRITT-BERICHTE VDI, Bd. 17, Nr. 259, 2005, Seiten 116-153, XP002583667 Düsseldorf ISSN: 0178-9600 ISBN: 3-18-325917-6
- Ulrich Moissl: "Kardiovaskuläre Überwachung bei der Hämodialysetherapie", Fortschritt-Berichte VDI, vol. 17, no. 259, 1 January 2005 (2005-01-01), pages I-153, XP055126320, Düsseldorf ISBN: 978-3-18-325917-5

## Beschreibung

Die vorliegende Erfindung betrifft eine Vorrichtung zum Durchführen eines Verfahrens zum Ermitteln und/oder Überwachen eines körperlichen Zustandes eines Patienten gemäß Anspruch 1 und eine Blutbehandlungsvorrichtung gemäß Anspruch 6.

Zum Ermitteln und Überwachen des körperlichen Zustandes eines Patienten werden in der Praxis geeignete Größen ermittelt und ausgewertet. Basierend hierauf können Rückschlüsse auf den Zustand des Patienten zu einem vergangenen Zeitpunkt, zum Zeitpunkt einer Messung oder zu einem späteren Zeitpunkt gezogen werden; gegebenenfalls werden entsprechend Maßnahmen ergriffen. Derartige Verfahren und dazu verwendete Vorrichtungen sind für die Herzrate - als Beispiel für eine ermittelte und ausgewertete Größe - aus der EP 0 330 761 A1 und der EP 0 957 956 bekannt.

In "New Blood Volume Monitoring Device for Hemodialysis: A-V Pressure Gradient Measurement by Syncronized One-point Reading" erschienen in Artificial Organs 28, 7 (2004) stellen Masami Oda et al. ein Verfahren vor, um eine einfache Darstellung des Blutvolumens zu ermöglichen.

Im Aufsatz "Ansätze zur Identifikation von Patientenparametern während der Hämodialysetherapie" erschienen in AT - Automatisierungstechnik, 50 (2002) beschreiben Peter Wabel et al. ein Verfahren, um die Herzfrequenz abhängig vom Blutdruck zu messen.

In der US 2006/0074369 A1 ist eine Blutreinigungsvorrichtung offenbart.

In einem Paper der Work-Group "Vascular Access 2006" mit dem Titel "clinical practice guidelines for vascular access" erschienen im American Journal of Kidney diseases, 48 Suppl. 1 (2006-07) wird das Verhältnis des Drucks innerhalb des Zugangs mit dem durchschnittlichen arteriellen Druck verglichen, um Stenosen zu erkennen.

Aus der DE 199 01 078 C1 gehen ein Verfahren und eine Vorrichtung zur Erkennung von Stenosen bei der extrakorporalen Blutbehandlung hervor.

Es ist Aufgabe der vorliegenden Erfindung, eine weitere Vorrichtung zur Durchführung eines Verfahrens zum Ermitteln und/oder Überwachen eines körperlichen Zustandes eines Patienten anzugeben.

Die erfindungsgemäße Aufgabe wird durch eine Vorrichtung mit den Merkmalen des Anspruchs 1 gelöst. Vorteilhafte Weiterentwicklungen der vorliegenden Erfindung sind jeweils Gegenstand der Unteransprüche.

Die Vorrichtung umfasst eine Einrichtung, welche konfiguriert ist zum Auswerten einer Amplitude eines Drucksignals. Eine Amplitude eines Drucksignals ist dabei erfindungsgemäß als ein einzelner Wert - und insbesondere dessen Höhe - zu verstehen, nicht als ein Signal.

Ein Erfassen der Herzrate zur Beurteilung des körperlichen Zustands eines Patienten ist wie oben ausgeführt bereits bekannt. Nun hat sich überraschend bei Untersuchungen der Anmelderin von Amplituden von Drucksignalen von 50 Patienten, bei welchen eine Blutbehandlung durchgeführt wurde, herausgestellt, dass eine "Amplitude eines Drucksignals" in vorteilhafter Weise ebenfalls zum Beurteilen des körperlichen Zustands des Patienten verwendet werden kann. Dabei ist unter "Amplitude eines Drucksignals" eine am Körper eines Patienten oder extrakorporal - z. B. bei einer medizinischen Behandlung - gemessene oder aus solchen Messungen ermittelte Amplitude eines Drucksignals, insbesondere eine Amplitude eines Herzdrucksignals des Patienten oder eine Näherung hiervon, zu verstehen. Die Amplitude eines Drucksignals, und insbesondere die Amplitude eines Herzdrucksignals, bzw. deren Verlauf, kann ebenso wie die Herzrate Schwankungen bzw. Veränderungen, beispielsweise während einer Dialysebehandlung des Patienten oder auch über längere Zeiträume hinweg, unterworfen sein.

Bei einer Dialysebehandlung können die Ursachen hierfür Änderungen des Schlagvolumens des Herzens, des Fisteldrucks, der Füllung der Fistel beziehungsweise des Gefäßsystems, des Sitzes der Nadel, ein arterieller Unterdruck im Schlauchsystem der Blutbehandlungsvorrichtung, eine sich entwickelnde Zu- oder Abflussstenose der Fistel - insbesondere bei Goretex-Grafts - Gefäßsteifigkeit, Elastizität, Kalzifizierung der Fistel und/oder der zu-/abführenden Blutgefäße oder Reflexionen der Pulswelle im Gefäßsystem und andere sein.

Bei Dialyse-Patienten wie bei Nicht-Dialyse-Patienten können weitere Gründe zur Schwankung oder Veränderung führen oder diese begünstigen. Die Schwankungen der Amplitude des Drucksignals und insbesondere der Amplitude des Herzdrucksignals können durch das Schlagvolumen des Herzens, das Herzzeitvolumen, den Auswurf, die Kontraktilität, Herzklappenfehler, den Gefäßstatus - insbesondere bei Diabetespatienten - oder Änderungen hiervon bedingt sein. Die Schwankungen oder Veränderungen können ferner durch technische Gegebenheiten oder andere Umstände wie Punktionsort, Stärke der Nadel, Compliance eines verwendeten Systems, Lagewechsel des Patienten, Bewegungen des Patienten, Rezirkulation, hydrostatische Druckaufschläge, Änderungen des TPR (total peripheral resistance oder Gefäßwiderstand in peripheren Blutgefäßen) oder dergleichen bedingt sein. Die Auswertung der Amplitude des Drucksignals und vor allem der Amplitude des Herzdrucksignals kann in vorteilhafter Weise zum Beurteilen der oben genannten und weiterer Phänomene oder Veränderungen im Sinne eines "körperlichen Zustandes" - wie vorliegend zu verstehen - eingesetzt werden. Bei Dialysepatienten kann insbesondere die Abschätzung des Schlagvolumens oder Herzzeitvolumens (cardiac output) des Herzens mittels einer Auswertung der Amplitude des Drucksignals von großem Interesse sein.

Eine "Amplitude des Drucksignals" kann der ermittelte oder gemessene Wert des Drucksignals - oder dessen Höhe - in einer maximalen Auslenkung bzw. in einem Maximum bezeichnen. Die maximale Auslenkung kann als die Differenz zwischen dem Wert des maximalen Druck-Peaks und einer Grundlinie, üblicherweise einem Mitteldruck, wie beispielsweise 0 mmHg, bestimmt sein. Die Amplitude des Drucksignals kann ferner auf andere Weise festgelegt sein.

Das "Ermitteln und/oder Überwachen wenigstens eines körperlichen Zustandes" oder einer Größe den körperlichen Zustand eines Patienten betreffend (wobei "körperlicher Zustand" und "Größe, den körperlichen Zustand betreffend" im Folgenden auch synonym verwendet sein können) kann sowohl zum Beurteilen eines pathologischen Zustands als auch eines nicht krankhaften, insbesondere physiologischen, Zustands des Patienten erfolgen. Es kann zu dessen Überwachung und/oder zur Begleitung oder Überwachung einer Behandlung des Patienten und/oder zu diagnostischen Zwecken erfolgen.

Das Ermitteln und/oder Überwachen kann ohne Vergleich der Amplitude des Drucksignals oder der Auswertungsergebnisse mit Referenzwerten weiterer Patienten erfolgen. So kann das Verfolgen einer intra-individuellen Entwicklung oder Veränderung der Amplitude des Drucksignals stets desselben Patienten über einen Zeitraum bereits zu einer Aussage über dessen körperlichen Zustand führen. Ein Vergleich mit Referenzdaten Dritter ist hierzu ersichtlich nicht erforderlich, jedoch möglich.

Das "Ermitteln und/oder Überwachen" kann während einer Behandlung des Patienten erfolgen, es kann jedoch auch zu einem späteren Zeitpunkt ohne Beisein des Patienten beziehungsweise ohne dass der Patient noch an eine Einrichtung zum Ermitteln der Amplitude des Drucksignals und/oder einer Behandlungsvorrichtung angeschlossen ist, erfolgen. Ferner muss die Amplitude des Drucksignals nicht im engeren Sinn am Patienten ermittelt worden sein. Die Amplitude des Drucksignals kann auch extrakorporal an einer Blutpumpe, einem Blutkreislauf oder dergleichen ermittelt worden sein. Erfindungsgemäß wird das Drucksignal mittels eines Drucksensors auf der arteriellen Seite eines extrakorporalen Blutkreislaufs ermittelt.

Eine "Größe, den körperlichen Zustand eines Patienten betreffend" oder ein "körperlicher Zustand" kann jede beliebige, insbesondere physiologische, Größe sein, die als charakteristische Größe zum Beurteilen und Kennzeichnen des körperlichen Zustands - oder eines Teilaspekts hiervon - eines Patienten geeignet ist. Beispiele hierfür schließen kardiovaskuläre Größen wie Fisteldurchblutung bei einem Dialysepatienten mit Fistelanlage oder Shunt, eine Herzrate, eine Herzdruckamplitude, einen Fistelzustand, eine Arrhythmie, die Funktion eines Herzschrittmachers und respiratorische Größen ein, ohne jedoch hierauf beschränkt zu sein. Eine respiratorische Größe kann zum Beispiel ein Atmungssignal wie die Atemfrequenz, ein pathologisches Atemmuster wie eine paradoxe Atmung, und dergleichen sein. Unter dem körperlichen Zustand wird jedoch nicht bereits die Amplitude des Drucksignals und insbesondere nicht die Amplitude des Herzdrucksignals verstanden. Erst deren Auswertung, nicht bereits ihre Messung oder Ermittlung, erlaubt den Rückschluss auf den körperlichen Zustand. Erfindungsgemäß ist der körperliche Zustand eine sich entwickelnde Ablussstenose einer Fistel.

Obwohl die vorliegende Beschreibung an verschiedenen Stellen exemplarisch auf Dialysepatienten bezogen ist, ist die vorliegende Erfindung keinesfalls auf solche Dialysepatienten beschränkt.

Ein "Patient" im Sinne der vorliegenden Erfindung kann ein Zugehöriger jeder Spezies (Mensch oder Tier) sein, gleich ob krank oder gesund.

Das "Auswerten der Amplitude des Drucksignals" kann mit Hilfe entsprechender und ggf. unterschiedlicher Auswerteeinrichtungen und/oder Auswerteverfahren erfolgen. Dabei kann das "Auswerten der Amplitude des Drucksignals" sowohl ein exaktes Auswerten der Höhe oder des Verlaufs der Amplitude des Drucksignals - zum Beispiel mittels verschiedener Rechenoperationen - wie auch ein Abschätzen der Höhe oder des Verlaufs der Amplitude des Drucksignals beziehungsweise eines Trends desselben aus der ermittelten Amplitude des Drucksignals bzw. der Höhe oder des Verlaufs beinhalten. Die im Einzelfall erforderliche Auswertung kann von Patient zu Patient verschieden sein; ein Fachmann kann anhand der gegebenen Umstände bestimmen, welche Art der Auswertung zur optimalen Betreuung des Patienten geeignet scheint. Beispiele einer Auswertung werden unten stehend gegeben. Eine Zuordnung zu bestimmten Krankheitsbildern oder Symptomen ist zur Ausführung des Verfahrens nicht erforderlich und nicht vorgesehen. Das Verfahren dient vielmehr dem Erzielen von Kennzahlen, Parameterwerten und dergleichen. Dies gilt insbesondere auch für den Schritt des Auswertens.

Zum "Auswerten" der Amplitude des Drucksignals kann jede aus der Amplitude erkennbare oder herleitbare Information sowie aus den Mess- oder Bestimmungsbedingungen herleitbare Information verwendet werden. So kann bereits die Höhe der Amplitude eine Aussage erlauben. Auch eine Höhe der Amplitude verglichen mit Amplituden anderer Patienten kann eine Aussage erlauben; ihr Vergleich mit derartigen Daten und das Ziehen von Erkenntnissen hieraus kann daher ebenfalls ein Auswerten im Sinne der Erfindung sein. Ferner kann der Verlauf der Amplitude eine Information über den körperlichen Zustand geben. Dabei kann der Verlauf aus einer Darstellung mehrerer Herzamplitudenwerte über der Zeit entsprechen oder dieser entnommen werden. Eine Zeitdifferenz (=1/Abtastrate, entsprechend also dem Kehrwert der Abtastrate) zwischen einzelnen Herzamplitudenwerten kann z.B. die Hälfte desjenigen Zeitfensters (hier 5 Sekunden) betragen, über welches die Amplitude ermittelt wurde. Zum "Auswerten" kann auch die mittlere Amplitude des Druck- und insbesondere des Herzdrucksignals, beispielsweise während einer Behandlungssitzung des Patienten oder eines anderen Zeitraums, verstanden werden. Wurden beispielsweise über 10-Sekunden-Abschnitte während einer Behandlung quasi-stationäre Werte für die Amplituden des Drucksignals ermittelt, so kann man über diese Werte (d.h. also über deren Verlauf hinweg) den Mittelwert oder Median bilden, und erhält somit pro Behandlung - oder für einen Zeitraum allgemein - einen Wert, den man über einen längeren Zeitraum (Wochen/Monate) hinweg verfolgen kann. Auch durch Auswerten dieses Wertes sind Aussagen über den körperlichen Zustand möglich. Ferner kann es möglich sein, Trends zu bestimmen. Natürlich fällt unter "Auswerten" auch ein Vergleich eines Verlaufs der Amplitude mit Verläufen bei weiteren Patienten.

Bevorzugt kann die Amplitude des Drucksignals allein ausgewertet werden. Es können zum Ermitteln und/oder Überwachen jedoch auch zusätzliche Größen berücksichtigt werden. Dabei können die Größen zu gleichen oder verschiedenen Zeitpunkten gemessen werden. Die Größen können in direktem Zusammenhang miteinander stehen oder voneinander unabhängig sein.

Ferner kann eine solche Größe - wie auch die Amplitude des Drucksignals - auch ermittelt und/oder überwacht werden, indem sie nicht direkt als Messwert erfasst, sondern durch Auswerten, Filtern, Umrechnen oder dergleichen einer anderen, insbesondere gemessenen, Größe erhalten wird.

Die andere Größe kann dabei eine auf andere Weise zugängliche Größe sein. Die o. g. Auswertung kann beispielsweise durch Messen und Auswerten von zusätzlichen Größen wie Herzrate, mittlerem Fisteldruck ((Pₐᵣₜ (arterieller Fisteldruck) + Pᵥₑₙ (venöser Fisteldruck))/2) oder anderen, insbesondere dialysespezifischen Parametern, ergänzt werden.

Um aussagekräftige Rückschlüsse ziehen zu können, muss ein "Auswerten" nicht notwendigerweise exakte Werte liefern. Es kann ggf. auch ausreichen, eine Annäherung an die tatsächlich vorliegenden exakten Werte zu ermitteln. Auch hiermit kann sich ein Verlauf der ermittelten Werte und damit ein Trend derselben verfolgen lassen. Eine solche Vorgehensweise kann dazu dienen, den Aufwand für die Auswertung der ermittelten Größe weiter zu verringern. Sowohl der apparative Aufwand als auch ein zur Auswertung der Amplitude des Drucksignals erforderlicher Rechenaufwand können so vorteilhaft verringert werden.

Bei der Beschreibung der vorliegenden Erfindung kann der Begriff des "Drucksignals" als kontinuierliches Drucksignal - falls ausreichend hoch abgetastet (z.B. 20 Hz) - verstanden werden. Dieses Signal kann man in kleine Zeitabschnitte von z.B. 10 Sekunden unterteilen, in denen Frequenz und Amplitude als konstant betrachtet werden (es wird dann von einem stationären Signal gesprochen). Das Drucksignal kann aus unterschiedlichen Signalen zusammengesetzt sein. Ein Anteil des Drucksignals kann ein Herzdrucksignal sein, also ein durch die Herzkontraktion bewirktes Signal. Ein weiterer Anteil kann ein Pumpendrucksignal sein, also ein von einer Blutpumpe hervorgerufenes, ggf. kontinuierliches, Drucksignal.

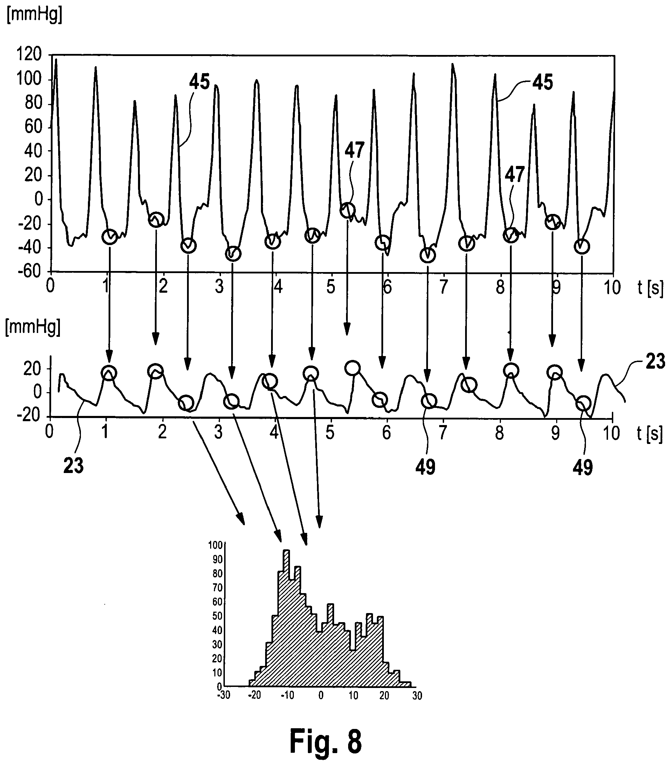

Bei einer "Amplitude des Drucksignals" - oder auch eines Herzdrucksignals - wird die Amplitude eines stationären oder annähernd stationären (quasi-stationären) (Herz-)Drucksignal-Abschnitts verstanden, beispielsweise über ein Zeitfenster von 10 Sekunden. Mit Hilfe eines Histogramms wie in Figur 8 gezeigt erhält man folglich eine Schätzung eines einzigen Herzamplitudenwertes über dasjenige Zeitfenster, welches zum Füllen des Histogramms verwendet wurde (das Zeitfenster kann beispielsweise eine Dauer zwischen 1 und 5 Minuten haben).

Die mittels des Verfahrens ausgewertete Amplitude des Drucksignals kann während einer Blutbehandlungssitzung unter Verwendung einer Blutbehandlungsvorrichtung ermittelt werden. Sie kann insbesondere extrakorporal ermittelt oder gemessen werden.

Eine "Blutbehandlungsvorrichtung" kann zur Blutbehandlung und/oder Blutreinigung eingesetzt werden und als eine Dialysevorrichtung oder eine Infusionspumpe ausgestaltet sein, welche beispielsweise mittels Shunt, Fistel oder Katheter mit dem Gefäßsystem des Patienten verbunden ist. Bei einer Dialysevorrichtung kann es sich u. a. um eine Einrichtung für eine Hämodialyse, Hämofiltration oder Hämodiafiltration handeln. Regelmäßig weisen derartige Einrichtungen eine extrakorporale Blutpumpe auf.

Um aussagekräftige Rückschlüsse ziehen zu können, muss ein "Ermitteln" nicht notwendigerweise exakte Werte liefern. Regelmäßig genügt es, lediglich ein Niveau oder einen Trend derselben zu kennen. Auch eine Annäherung an die tatsächlich vorliegenden exakten Werte kann ggf. genügen. Eine derart vereinfachte Vorgehensweise kann den Aufwand für das Ermitteln der relevanten Größe weiter verringern. Der apparative Aufwand zum Ermitteln der Größe, insbesondere ein Mess- oder Rechenaufwand, kann so vorteilhaft verringert werden.

Eine "Amplitude eines Drucksignals" kann im extrakorporalen Blutkreislauf mit einem oder mehreren der in einer Blutbehandlungsvorrichtung vorgesehenen Drucksensoren gemessen werden.

Geeignete Drucksensoren sind im Stand der Technik allgemein bekannt und schließen, ohne darauf beschränkt zu sein, piezoelektrische, piezoresistive, frequenzanaloge, kapazitative, induktive Drucksensoren und/oder Drucksensoren mit Hallelementen sowie Kombinationen derselben ein.

Ein solcher Drucksensor kann im arteriellen oder venösen Zweig des Schlauchsystems oder anderen Abschnitten, insbesondere an der Blutpumpe der Blutbehandlungsvorrichtung, integriert sein.

Neben der gewünschten Amplitude des Drucksignals, welche beispielsweise die tatsächliche Amplitude des Herzdrucks des Patienten ist, kann die Amplitude des Drucksignals weitere unerwünschte Drucksignale, wie beispielsweise Störsignale, die von weiteren in der Blutbehandlungsvorrichtung eingesetzten Einrichtungen stammen können, und/oder Messrauschen umfassen.

Diese für eine Auswertung ggf. unerwünschten weiteren Amplituden von Drucksignalen können mittels bekannter Verfahren vom relevanten Signalanteil eliminiert werden.

Das Messrauschen kann zum Beispiel über einen einfachen Bandpassfilter eliminiert werden.

Das Verfahren umfasst daher einen Schritt des Korrigierens des Wertes der Amplitude des Drucksignals um einen Beitrag der Blutbehandlungsvorrichtung zur Höhe der Amplitude des Drucksignals.

Ein solcher Beitrag kann zum Beispiel eine von einer bei der Blutbehandlung verwendeten extrakorporalen Blutpumpe stammende Amplitude des Drucksignals bzw. dessen Höhe sein.

Das "Korrigieren der Amplitude des Drucksignals" kann gemäß den aus der EP 0 330 761 A1 und der EP 0 957 956 B1 bekannten Verfahren erfolgen.

Daneben kann auch das aus der Dissertation einer der Erfinder der vorliegenden Erfindung, Ulrich Moissl, mit dem Titel "Kardiovaskuläre Überwachung bei der Hämodialysetherapie", Technische Universität Darmstadt, Deutschland, 2005, bekannte Verfahren verwendet werden. Gemäß der dort beschriebenen Vorgehensweise wird die Amplitude des Herzdrucksignals während der extrakorporalen Blutbehandlung des Patienten in exemplarischen Abständen von jeweils 10 Sekunden unter Berücksichtigung eines aktuellen Signalmodells der Blutpumpe ermittelt. Dies erlaubt eine realitätsnahe Nachbildung des zeitlichen Verlaufs und der Amplitude des Drucksignals. Ein Online-Verfahren ist in der Dissertation ebenfalls beschrieben, welches bei jedem neuen Abtastpunkt (also z.B. 20 Mal pro Sekunde) den neuen Herzdrucksignalwert ausgibt. Die hierzu verwendeten Algorithmen sind in der oben genannten Dissertation beschrieben.

Aus der DE 101 15 991 C1, der EP 0 330 761 A1, der EP 0 957 956 B1 und der US 2005/0010118 A1 sind Verfahren zur Trennung von Herzsignal und Blutpumpensignal bekannt, bei welchen die Signale aufgrund unterschiedlicher Frequenzspektren getrennt werden.

Das Korrigieren der Höhe der Amplitude des Drucksignals kann zeitgleich mit der Messung desselben oder zu einem späteren Zeitpunkt erfolgen.

Es kann zum Beispiel automatisch zu bestimmten vorgegebenen Zeitpunkten erfolgen.

Die Abstände zwischen den Zeitpunkten können in Abhängigkeit von äußeren Bedingungen oder von Messergebnissen variieren.

Eine bevorzugte Ausführungsform des Verfahrens sieht das Korrigieren des Wertes der Amplitude des Drucksignals in Abhängigkeit von einem von wenigstens einem Positionssensor übermittelten Signal vor. Der Positionssensor ermöglicht eine Abtastung des Herzdrucksignals zu einem konstanten Drehwinkel des Pumpenrotors.

Der Positionssensor kann ein Hallsensor sein oder mit diesem zusammenwirken, welcher einen Impuls abgibt, aber auch als ein optischer Sensor, der z.B. einen schwarzen Strich am Rotor erkennt, oder auf jede andere, dem Fachmann bekannte Weise, ausgestaltet sein. Ein "Hallsensor" ist eine Einrichtung zum Messen einer elektrischen Spannung in einem stromdurchflossenen Leiter, der sich in einem stationären Magnetfeld befindet. Die Funktionsweise von Hallsensoren ist zum Beispiel in der Abhandlung von Josef Janisch, "Was Sie schon immer über Hallsensoren wissen wollten: Kleiner Effekt - Große Wirkung", Seiten 1 bis 5, elektronik industrie 7, 2006, beschrieben.

Der oder die Hallsensoren können eingesetzt werden wie in der DE 102 30 413 A1 beschrieben.

Das "Korrigieren des Wertes der Amplitude des Drucksignals in Abhängigkeit von einem von einem Positionssensor, insbesondere einem Hallsensor übermittelten Signal oder abgegebenem Impuls" bedeutet beispielsweise, dass die Amplitude des ermittelten Drucksignals um einen zu einem bestimmten Zeitpunkt vom einem Sensor aufgrund eines vom Hallsensor erzeugten Impulses übermittelten Beitrag der Blutbehandlungsvorrichtung zur Höhe der Amplitude des Drucksignals korrigiert wird.

Ein solcher Beitrag kann eine Höhe einer von der Blutpumpe stammenden Amplitude des Drucksignals sein. Andere Berücksichtigungen des Hallsensorsignals sind natürlich ebenfalls möglich. Dies gilt insbesondere für dem Fachmann bereits bekannte Verfahren.

Ein Abtasten erfolgt bei dieser Ausführungsform bei jeder Pumpenumdrehung jeweils in derselben Rotorstellung. Auf diese Weise wird das Drucksignal hinsichtlich der relevanten Herzsignalanteile effektiv unterabgetastet. Die Amplitude des Herzdrucks lässt sich hierbei aufgrund der Unterabtastung nicht kontinuierlich berechnen, jedoch erhält man ausreichend viele Werte des bspw. über Minuten hinweg als konstant betrachteten Verlaufs des Drucksignals und insbesondere des Herzdrucksignals.

Die Amplitude, welche den Maximalwert des Drucksignals darstellt, kann aus den ermittelten Drucksignalwerten geschätzt werden. Wird stets zu einem Höchstwert des Drucksignals ermittelt, so erhält man dadurch implizit sogar dessen Amplitude.

Vor allem bei ihrer entsprechenden Darstellung - z. B. mittels Histogramms - erlauben sie eine gute Schätzung der Amplitude. Da derartige Werte mehrmals während einer Blutbehandlungssitzung - und ferner über mehrere solcher Sitzungen hinweg - erhoben werden können, stellt z. B. ihre Standardabweichung - und ggf. deren Mittelung oder anderweitige geeignete mathematische Bearbeitung - eine gute Schätzung für bspw. die mittlere Amplitude über die Behandlungsdauer dar. Insbesondere sind hierbei Trends - also längerfristige Entwicklungen - gut erkennbar.

Wenn die Pumpe ausreichend gleichmäßig läuft und die Umdrehungsdauer ausreichend exakt bekannt ist, so lässt sich das Histogramm auch durch eine zeitsynchrone Abtastung aufbauen.

In einer weiteren bevorzugten Ausführungsform umfasst das Verfahren das Korrigieren des ermittelten Wertes der Amplitude des Drucksignals in Abhängigkeit von einem Drehwinkel einer - insbesondere peristaltischen - Blutpumpe der Blutbehandlungsvorrichtung. Eine "peristaltische Blutpumpe" ist eine üblicherweise in einem Blutbehandlungs- und/oder Blutreinigungsverfahren eingesetzte Verdrängerpumpe zum Fördern des Blutstroms im extrakorporalen Blutkreislauf. Sie kann zum Beispiel als Rollenpumpe ausgebildet sein.

Das "Korrigieren des Wertes der Amplitude des Drucksignals in Abhängigkeit von einem Drehwinkel einer peristaltischen Blutpumpe" bedeutet, dass die Amplitude des Drucksignals um ein bestimmtes Signal der Blutpumpe, das mit einem bestimmten Drehwinkel der Blutpumpe korreliert, korrigiert wird. Das heißt, dass die jeweiligen Amplituden bei einem bestimmten Drehwinkel der Blutpumpe gemessen und/oder unter Berücksichtigung des Drehwinkels korrigiert werden. Dies bietet sich insbesondere bei nicht ausreichend gleichmäßig rotierenden Blutpumpen vorteilhaft an. Dies ist vor allem bei der winkelsynchronen Abtastung vorteilhaft möglich.

Erfindungsgemäß umfasst das Verfahren das Auswerten des Wertes der korrigierten Amplitude des Drucksignals durch Vergleichen der Amplitude mit vorgegebenen Referenzwerten.

Die "vorgegebenen Referenzwerte" können von demselben Patienten stammen und/oder von anderen Patienten erhaltene Werte und/oder Erfahrungswerte und/oder Werte von in pathologischer Hinsicht unauffälligen Personen sein.

Eine solche Auswertung kann anhand des Vergleichs exakter Werte erfolgen. Es kann jedoch ebenso ausreichend sein, einen bestimmten Trend der korrigierten Amplitude des Drucksignals bzw. deren Höhe in Bezug auf die bekannten Referenzwerte zu betrachten.

Auf Basis einer entsprechenden Auswertung kann es zum Beispiel möglich sein, einen zum Zeitpunkt des Auswertens aktuellen körperlichen Zustand des Patienten zu erfassen und auf Basis der erhaltenen Ergebnisse Rückschlüsse auf mögliche kritische oder eine Handlung erfordernde Situationen zu ziehen und gegebenenfalls rechtzeitig Maßnahmen einzuleiten.

Ein wiederholtes Auswerten der Amplitude kann ferner dazu dienen, einen Behandlungsverlauf abzubilden oder einen Behandlungserfolg nachzuweisen.

Das Verfahren, zu dessen Durchführung die Vorrichtung der vorliegenden Erfindung konfiguriert ist, umfasst das extrakorporale Ermitteln oder Messen der Amplitude des Drucksignals. Bevorzugt wird das Verfahren als offline-Verfahren durchgeführt, so dass der Patient in vorteilhafter Weise für das Ermitteln oder das Auswerten nicht weiter anwesend sein muss. Er muss zur Auswertung beispielsweise auch nicht an eine Behandlungsvorrichtung angeschlossen bleiben.

Aber auch eine online-Durchführung des Verfahrens sowie Kombinationen aus online (z.B. für die Datenerhebung) und offline (z.B. für die Datenauswertung) ist von der Erfindung umfasst.

In einer Ausführungsform des Verfahrens, welche nicht unter die vorliegende Erfindung fällt, ist die überwachte oder ermittelte Größe ein Atmungssignal.

Das Atmungssignal kann bei Patienten mit einem Herzkatheter durch Messungen im rechten Vorhof des Patienten ermittelt werden und beispielsweise die Atemfrequenz und/oder die Atemtiefe eines oder mehrerer Atemzüge betreffen.

Durch das Ermitteln und/oder Überwachen des Atmungssignals basierend auf der Kenntnis der Amplitude des Drucksignals und insbesondere der Amplitude des Herzdrucksignals kann zum Beispiel ein Atmungsprofil des Patienten abgebildet und so Atmungsschwankungen beziehungsweise eine ungleichmäßige Atmung, wie beispielsweise eine Cheyne-Stokes-Atmung, erfasst werden.

Ebenso ist es möglich, mittels des Verfahrens eine Schlaf-Apnoe zu erkennen.

Das Verfahren umfasst ferner ein Auswerten der Amplitude des Drucksignals zum Beobachten eines Langzeit-Trends von, insbesondere kardiovaskulären, Größen.

Das "Beobachten des Langzeit-Trends" kann dabei einen Zeitraum von mehreren wenigen Stunden bis einigen Wochen und/oder Monaten umfassen. Ein solcher Zeitraum umspannt beim Dialysepatienten eine Mehrzahl von Dialysebehandlungen.

Es kann somit möglich sein, einen zeitlichen Verlauf des körperlichen Zustands eines Patienten abzubilden. So kann beispielsweise eine Fistel und insbesondere eine Fistelneuanlage über mehrere Monate unter Auswertung des Druckamplitudensignals überwacht werden, um Veränderungen am Gefäßzugang zu erkennen, bevor diese kritisch bzw. behandlungsbedürftig werden. Auf diese Weise kann es in vorteilhafter Weise möglich sein, aufwendige und teils teure Eingriffe bzw. Fistelneuanlagen zu vermeiden. Beginnende Stenosen können ggf. rechtzeitig erkannt und dilatiert werden, beispielsweise unter Einsatz von Ballonkathetern.

Zu diesem Zweck wird die Amplitude des Herzdrucksignals des Patienten - ggf. zusätzlich zu einer Veränderung der Herzrate, des mittleren Fisteldrucks oder einer Veränderung hiervon und/oder anderen dialysespezifischen Variablen oder deren Veränderung - in einer Langzeitüberwachung erfasst.

Das Verfahren kann während einer Blutbehandlung, insbesondere einer Hämodialyse, einer Hämofiltration oder einer Hämodiafiltration, eingesetzt werden.

Dabei kann es von Vorteil sein, die bereits für die Blutbehandlung vorgesehenen Mess- und/oder Auswerteeinrichtungen der Blutbehandlungsvorrichtung zum Durchführen des Verfahrens einzusetzen.

In einem Ausführungsbeispiel, das nicht unter die vorliegende Erfindung fällt, kann das Verfahren zum Auswerten der ermittelten Amplitude des Drucksignals auf Tachykardie, Bradykardie und/oder hyper- oder hypotensive Episoden eingesetzt werden.

In einer Weiterbildung, welche nicht unter die vorliegende Erfindung fällt, dient das Verfahren zum Erstellen einer Klassifikation eines Patienten mit den hierzu bekannten Vorteilen. Insbesondere kann durch Klassifikation die Genauigkeit der Aussage für einen individuellen Patienten erhöht werden.

In weiteren Ausführungsformen, welche nicht unter die Erfindung fallen, ist die zu ermittelnde und/oder überwachende Größe eine Herzrate und/oder eine Arrhythmie und/oder die Funktion eines Herzschrittmachers. Durch Ermitteln und/oder Überwachen einer Arrhythmie ist es beispielsweise möglich Extrasystolen oder Synkopen zu erkennen.

So ist es zum Beispiel möglich, eine Dialysatkonzentration in geeigneter Weise anzupassen, indem beispielsweise deren Kaliumgehalt erhöht oder deren Zusammensetzung anderweitig verändert wird.

Es kann ein Fourier-Spektrum der Herzamplituden (nicht zu verwechseln mit dem Herzdrucksignal) über eine Behandlung oder einen Behandlungsausschnitt (z.B. 10 bis 30 Minuten) gebildet werden. Hierdurch können rhythmische Veränderungen der Herzsignalamplitude erfasst werden. Aus Sicht eines kontinuierlichen 20 Hz-Herzdrucksignals wäre das eine Amplitudenmodulation. Man könnte hier in Analogie zur "Heart Rate Variability" bspw. von einer "Heart Amplitude Variability" sprechen.

Dieses Vorgehen könnte z.B. den Einfluss der hormonellen Blutdruckregelung des Barorezeptorregelkreises oder ähnlicher langfristiger Regelkreis-Oszillationen widerspiegeln, aber auch eine kurzfristige beat-to-beat Modulation des Blutangebots in der Fistel falls Herz und Pumpe (Rollenpumpen ziehen pulsatil und nicht kontinuierlich) nicht synchron "schlagen" bzw. fördern.

Dabei ist es vorstellbar, dass zunächst die Pumpe Blut zieht und erst Millisekunden später der nächste Blutschwall in die Fistel einläuft.

Bei Interesse können auch weitere, hier nicht genannte, insbesondere physiologische, Größen ermittelt und/oder überwacht werden.

Die erfindungsgemäße Vorrichtung ist in Anspruch 1 definiert. Die Vorrichtung kann jeweils die zur Durchführung des Verfahrens in jeder seiner Ausführungsformen erforderlichen und geeigneten Einrichtungen aufweisen. Zur Vermeidung von Wiederholungen der Funktion der einzelnen Komponenten, Elemente und/oder Vorteile wird auf die in Zusammenhang mit dem Verfahren erläuterten Komponenten, Elemente und/oder Verfahrensschritte verwiesen. Mittels der erfindungsgemäßen Vorrichtung lassen sich die mittels des Verfahrens erzielbaren Vorteile ungeschmälert erzielen.

Die Vorrichtung weist wenigstens entsprechende Einrichtungen zum Auswerten und ggf. Messen einer Amplitude eines Drucksignals auf.

Die Einrichtungen der Vorrichtung können automatisierte Einrichtungen und/oder solche zur Datenverarbeitung, wie beispielsweise eine CPU, sein oder aufweisen.

Die Amplitude des Drucksignals kann mittels entsprechender Einrichtungen statistisch ausgewertet werden. Sie kann wie die Herzfrequenz mittels entsprechender Einrichtungen während einer Dialysebehandlung aus der am extrakorporalen Blutkreislauf gemessenen Amplitude des Drucksignals extrahiert und ggf. aufgezeichnet werden.

Die erfindungsgemäße Vorrichtung umfasst ferner wenigstens eine Einrichtung zum Korrigieren des Wertes der Amplitude des Drucksignals um einen Beitrag einer Blutbehandlungsvorrichtung zur Höhe der Amplitude des Drucksignals, bevorzugt in Abhängigkeit eines Signals oder Impulses wenigstens eines Positionssensors oder Hallsensors und/oder in Abhängigkeit des Drehwinkels der einer anderen geeigneten technischen Größe einer Blutpumpe der Blutbehandlungsvorrichtung.

Ein solcher Beitrag kann eine Amplitude des Drucksignals einer Blutpumpe sein. Das Korrigieren der Amplitude des Drucksignals erfolgt vorzugsweise automatisch.

Der Zeitpunkt zum Erfassen des Beitrags der Blutbehandlungsvorrichtung zur Höhe der Amplitude des Drucksignals kann entsprechend durch ein bestimmtes Signal wenigstens eines Hallsensors und/oder einen bestimmten Drehwinkel einer Blutpumpe der Blutbehandlungsvorrichtung vorgegebenen sein.

Bei der Hallsensor-synchronen Abtastung kann das Pumpensignal bereits implizit korrigiert werden. Der Druckbeitrag der Pumpe ist im optimalen Fall immer gleich und beträgt z. B - 180 mmHg. Dieser Wert beinhaltet auch den mittleren Fisteldruck. Da dieser nicht genau bekannt ist, kann das Histogramm auf 0 normiert werden.

Ist der Beitrag der Blutpumpe allerdings bekannt, so kann hieraus auch der Fisteldruck berechnet werden: Bei identischer oder vergleichbarer Geometrie von Pumpe, Nadel und Schlauch usw. und bei gegebener Blutviskosität/gegebenem Hämatokrit lässt sich im Labor eine reine Pumpendruckkurve aufzeichnen.

Die Differenz zwischen deren Verlauf und einem Verlauf der Messwerte bzw. des Drucksignals ist der Fisteldruck, sofern alle anderen Bedingungen der Laborumgebung entsprechen; ansonsten lässt sich durch das o.g. Vorgehen eine Näherung des Fisteldrucks bzw. dessen Verlaufs erhalten.

In einer Weiterbildung der vorliegenden Erfindung weist die erfindungsgemäße Vorrichtung ferner eine Einrichtung zum Bereithalten von Referenzwerten auf.

Diese Einrichtung kann zum Speichern der Referenzwerte dienen und eine im Stand der Technik übliche und für diesen Zweck geeignete Speichereinrichtung, wie beispielsweise ein ROM, ein RAM, eine Diskette, eine Speicherkarte, ein USB-Stick und dergleichen sein.

Die erfindungsgemäße Vorrichtung kann ferner weitere Einrichtungen zum Filtern von Störsignalen und/oder zum Analysieren und/oder Umwandeln der Amplitude des ermittelten Drucksignals zum Erhalten des gewünschten Signals des Patienten aufweisen.

Die Filterung kann dabei wie in der o. g. Dissertation beschrieben erfolgen.

Die erfindungsgemäße Aufgabe wird auch mit einer Blutbehandlungsvorrichtung gemäß Anspruch 6 gelöst. Eine solche Blutbehandlungsvorrichtung umfasst wenigstens eine erfindungsgemäße Vorrichtung, wie sie vorstehend beschrieben ist. Eine Blutbehandlungsvorrichtung kann zum Beispiel als eine Dialysevorrichtung ausgestaltet sein, wie sie eingangs im Zusammenhang mit einer Blutbehandlung beschrieben wurde.

Mit dem Verfahren und der beschriebenen Vorrichtung ist es in vorteilhafter Weise möglich, wenigstens eine, insbesondere kardiovaskuläre, Größe, den körperlichen Zustand eines Patienten betreffend, zu ermitteln oder zu überwachen. Dies kann ohne besonderen Aufwand und zudem nicht-invasiv erfolgen. Seine Ausführung ist ferner ohne nennenswerten zusätzlichen apparativen Aufwand insbesondere während einer Blutbehandlung möglich. Das Verfahren erlaubt vorteilhaft insbesondere eine Veränderung an einem Gefäßzugang (z. B. Fistel oder Shunt) zu erkennen. Die gilt besonders bei einer Langzeitüberwachung der Amplitude des Drucksignals. Zum Ausführen des Verfahrens und/oder zum Verwenden der erfindungsgemäßen Vorrichtung ist vorteilhaft eine spezielle Ausbildung und/oder Schulung des Klinikpersonals und/oder des Personals in Arztpraxen nicht erforderlich. Das Verfahren zeichnet sich daher durch seine einfache Ausführbarkeit und vergleichsweise einfache Auswertbarkeit aus. Dies gilt insbesondere beim Messen der Amplitude des Drucksignals zu einem bestimmten vorgegebenen Zeitpunkt eines Hallsensorsignals und/oder drehwinkelsynchron zu der in der Blutbehandlungsvorrichtung vorgesehenen Blutpumpe und Korrigieren der ermittelten Amplitude des Drucksignals um einen bekannten Beitrag der Blutbehandlungsvorrichtung zum Signal, zum Beispiel um den Beitrag der Blutpumpe. Wenn die Amplitude des Drucksignals der Blutpumpe bei einem bestimmten Drehwinkel bekannt ist, kann das Korrigieren vorteilhaft weiter vereinfacht werden, da jeweils ein im Wesentlichen gleicher Betrag für den Wert des Beitrags der Blutpumpe zur Höhe der Amplitude angenommen und die ermittelte Amplitude des Drucksignals jeweils um diesen bekannten Wert korrigiert werden kann, um die gewünschte Amplitude des Herzsignals des Patienten oder einen Näherungswert zu erhalten. Dies kann insbesondere dann von Vorteil sein, wenn die Blutpumpe nicht ausreichend gleichmäßig rotiert. Die Möglichkeit, Kalzifizierungen innerhalb einer Fistel oder im Gefäßsystem des Patienten zu erkennen ist eine weitere vorteilhafte Anwendungsmöglichkeit der vorliegenden Erfindung.

Selbst wenn die Amplitude des Drucksignals der Blutpumpe nicht bekannt ist, kann die Amplitude des Herzdrucksignals bestimmt werden, falls der Beitrag der Pumpe immer derselbe ist (was durch die Hallsensor-synchrone Abtastung an immer gleicher Rotorstellung gewährleistet ist) oder zumindest näherungsweise derselbe ist.

Erfolgt die Unterabtastung zu einem Zeitpunkt, an dem sich das Pumpensignal nicht stark ändert, also die Ableitung nahe 0 ist (z.B. unmittelbar vor Eingriff einer der beiden Pumpenrollen in das Schlauchsystem bei einer Rollenpumpe), so erfordert die Messung keinen oder einen nur geringen technischen Aufwand. Ein solches Vorgehen kann durch günstiges Platzieren des Hallsensormagneten auf dem Rotor ermöglicht werden. Der Einfluss des Pumpensignals wird dabei vorteilhaft weiter minimiert.

Das Auswerten der Amplitude des Drucksignals, der Amplitude des Herzdrucksignals eines Patienten oder eines Näherungswertes hiervon kann in vorteilhafter Weise zum Lokalisieren von Stenosen und/oder zum Beurteilen des Zustands einer Fistel herangezogen und zum rechtzeitigen Verhindern kritischer Zustände verwendet werden. Durch Vergleichen der gewünschten charakteristischen Größen mit Referenzwerten und/oder Beobachten eines Trends der Größen kann der Entwicklung eines pathologischen Zustands vorgebeugt und/oder die Verschlechterung zu einem kritischen Zustand verhindert werden. Das Ausführen des Verfahrens bereitet einem Patienten keine Unannehmlichkeiten, insbesondere wenn das Verfahren - welches auch online einsetzbar ist - offline eingesetzt wird, was einen Vorteil der offline-Durchführung darstellt. Ein weiterer Vorteil des Verfahrens besteht darin, dass der Patient zum Zeitpunkt der Auswertung nicht anwesend sein muss. Die Kombination des Verfahrens mit einer Blutbehandlung, wie beispielsweise einer Hämodialyse, Hämofiltration oder Hämodiafiltration, sowie die Kombination der erfindungsgemäßen Vorrichtung mit einer für diesen Zweck geeigneten Blutbehandlungsvorrichtung erfordert kaum apparativen Aufwand und kann damit sowohl Zeit und Kosten einsparen als auch weitere Unannehmlichkeiten für den Patienten vorteilhaft vermeiden.

Ebenso ist es vorteilhaft möglich, nicht nur die direkt gemessenen Größen zum Beurteilen eines Zustands auszuwerten, sondern auf Basis der direkt gemessenen Größen Rückschlüsse auf andere Größen von Interesse zu ziehen. So existiert beispielsweise ein Zusammenhang zwischen Fisteldruck und Pulsamplitude. Nimmt zum Beispiel die Pulsamplitude während einer Behandlung ab, kann dies zum einen am Rückgang des Schlagvolumens, zum anderen an einer Abnahme des Fisteldrucks liegen. Es kann entsprechend möglich sein, den Effekt des Schlagvolumens über die erfasste Herzrate rechnerisch zu berücksichtigen und so auf eine relative Änderung des Fisteldrucks zu schließen. Ferner kann es möglich sein, über die Bestimmung der Amplitude des Drucksignals bei verschiedenen Drehzahlen der Blutpumpe auf das Blutangebot der Fistel zu schließen und somit gegebenenfalls den Fistelfluss abzuschätzen.

Obwohl das Verfahren oben auch im Zusammenhang mit einer Blutbehandlungsvorrichtung beschrieben ist, ist die vorliegende Offenbarung nicht auf eine Verwendung bei einem Patienten beschränkt, bei dem eine Blutbehandlung und/oder Blutreinigung mittels einer entsprechenden Vorrichtung durchgeführt wird.

Unter Bezugnahme auf die beiliegenden Zeichnung wird die vorliegende Erfindung beispielhaft beschrieben. Es gilt:
- Fig. 1: zeigt schematisch das Prinzip einer Herzsignal-Extraktion;
- Fig. 2: zeigt Graphen einer Herzrate (oben) und einer Amplitude eines Herzdrucksignals (unten) über der Zeit;
- Fig. 3: zeigt Wertebereiche von Amplituden des Herzdrucksignals eines Patienten über der Zeit, angegeben für die Monate Februar (02/08) bis September (09/08) des Jahres 2008;
- Fig. 4: zeigt Graphen einer Herzfrequenz (oben) und einer Amplitude eines Herzdrucksignals (unten) über der Zeit;
- Fig. 5: zeigt einen Graphen, der eine Überlagerung von Einflüssen der Atmung und der Amplitude des Herzdrucksignals über der Zeit wiedergibt;
- Fig. 6: zeigt einen Graphen, dem eine Cheyne-Stokes-Atmung zu entnehmen ist;
- Fig. 7: zeigt weitere Graphen einer Herzfrequenz (oben) und einer Amplitude eines Herzdrucksignals (unten) über der Zeit;
- Fig. 8: zeigt schematisch die Extraktion eines Herzdrucksignals (oben) und eine entsprechende Darstellung im Histogramm (unten);
- Fig. 9: zeigt schematisch das Abweichen einer Pumpenfrequenz von einer Sollfrequenz (Fig. 9a) sowie den Unterschied zwischen einer zeitsynchronen (Fig. 9b)und einer winkelsynchronen (Fig. 9c) Abtastung des Pumpensignals; und
- Fig. 10: zeigt einen Graphen einer Amplitude eines Herzdrucksignals über einem korrigierten Fisteldruck.

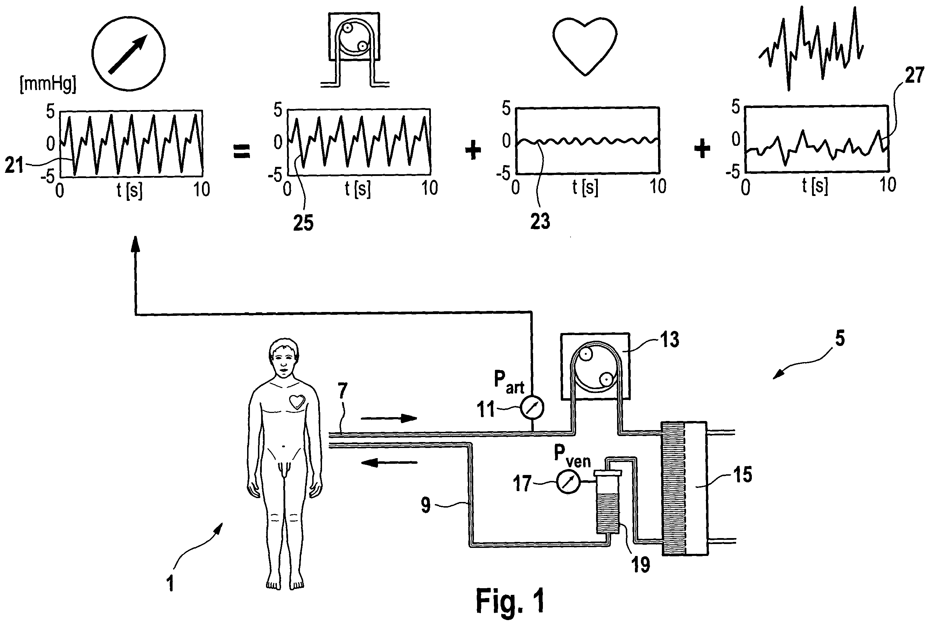

Fig. 1 stellt schematisch vereinfacht das Prinzip einer Herzsignal-Extraktion dar. Bei einem Patienten 1 wurde zum Zweck einer Blutbehandlung eine Fistel (nicht gezeigt) angelegt. Die Fistel ist mit einer Blutbehandlungsvorrichtung 5 verbunden, welche einen arteriellen Schenkel 7 und einen venösen Schenkel 9 aufweist. Die Blutbehandlungsvorrichtung 5 weist einen Dialysator 15, und auf ihrer arteriellen Seite einen Drucksensor 11, eine Blutpumpe 13, auf ihrer venösen Seite einen Drucksensor 17 und eine Tropfkammer 19 auf. Wie in Fig. 1 oben gezeigt ist, weist das mit dem Drucksensor 11 erfasste Drucksignal 21 das Herzdrucksignal 23 des Patienten 1, einen Beitrag 25 der Blutpumpe 13 und ein Messrauschen 27 auf. Die Amplitude des Herzdrucksignals 23 kann gemäß der vorliegenden Erfindung aus dem erfassten Drucksignal 21 ermittelt werden. Eine Auswertung der Amplitude des ermittelten Herzdrucksignals 23 ist wie oben beschrieben ebenfalls Gegenstand der vorliegenden Erfindung.

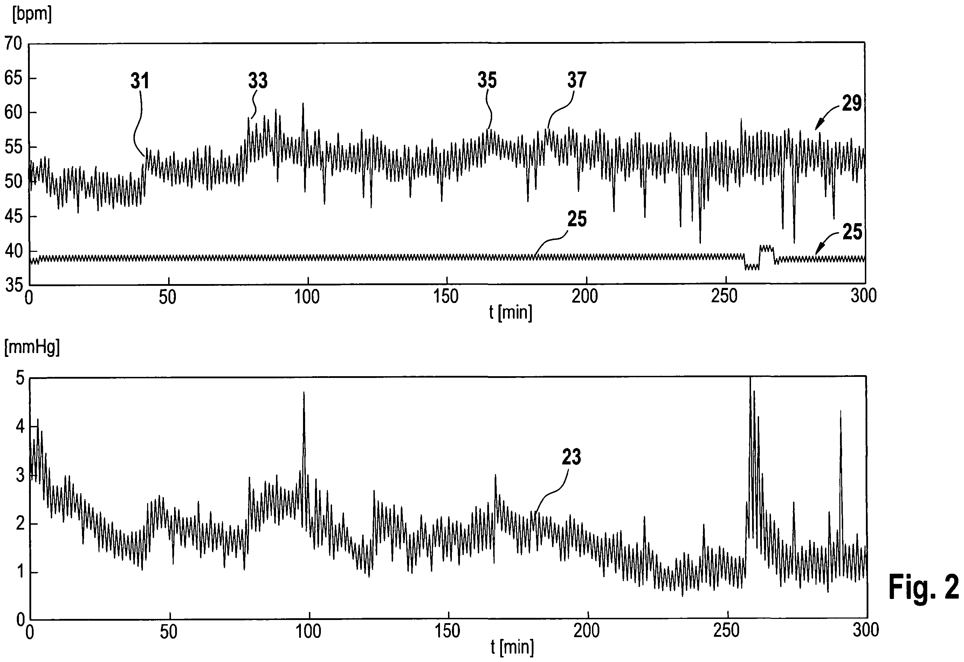

Fig. 2 zeigt Graphen einer Herzrate 29 (oben, angegeben in [bpm], also Schlägen pro Minute) und einer Amplitude eines Herzdrucksignals 23 (unten) über die Dauer einer Blutbehandlung. Die Herzrate wurde von den Erfindern mittels eines herkömmlichen EKG-Geräts validiert. Zusätzlich ist in Fig. 2 die Grundfrequenz 25 der Blutpumpe der verwendeten Blutbehandlungsvorrichtung mit der Bezeichnung 5008 des Unternehmens Fresenius Medical Care dargestellt. Der Verlauf der Herzrate 29 weist in Fig. 2 einige Sprünge auf. So steigt die Herzrate 29 erwartungsgemäß zum Zeitpunkt 31 des Aufwachens des Patienten, zum Zeitpunkt 33 des Frühstücks, zum Zeitpunkt 35 bei Erreichen der Halbzeit der Behandlung oder bei einer Visite zum Zeitpunkt 37 an. Das Herzdrucksignal 23 ist ebenso deutlichen Trends unterworfen. Seine Amplitude schwankt zwischen annähernd 4 und 1 mmHg.

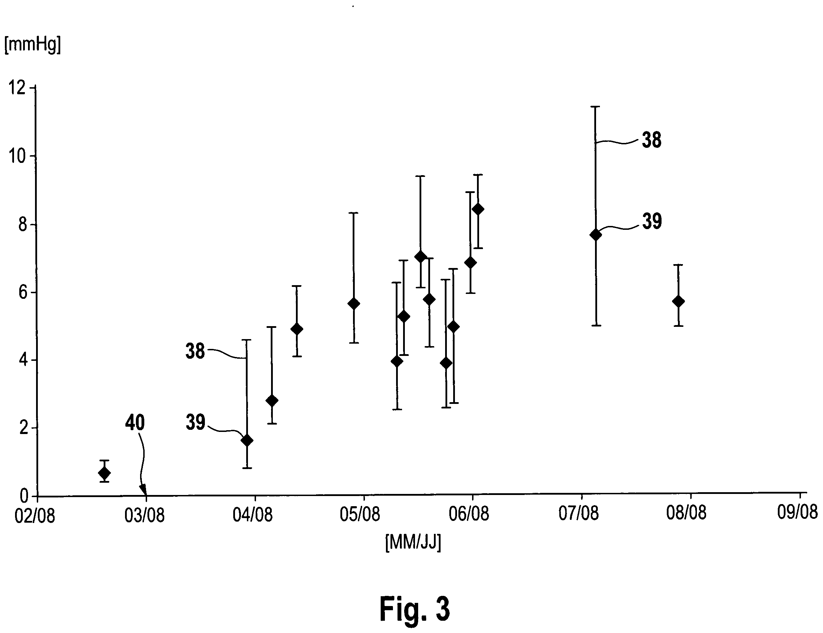

Fig. 3 zeigt Werte für die Amplituden eines Herzdrucksignals in mmHg über einer Zeit zwischen Februar (02/08) und September (09/08) des Jahres 2008. Die Balken 38 stellen jeweils den Median 39 sowie das zehnte und neunzigste Perzentil der Amplituden des Herzdrucksignals einer kompletten Behandlung dar. Nach der ersten Messung im Februar 2008 erfolgte eine Fistelneuanlage zu einem Zeitpunkt 40 als Graft (zuvor wurde ein zentralvenöser Katheter verwendet). Die mittlere Amplitude des Herzdrucksignals stieg daraufhin über Wochen hinweg stets weiter an, was auf eine sich entwickelnde Abflussstenose der Fistel hindeutet. Solche Abflussstenosen treten regelmäßig bei Goretex-Grafts auf. Der hier beobachtete Zeitraum stellt ungefähr die Reifungszeit für den Graft dar. Es kann ein Bereich definiert werden, in dem die Amplitude des Herzdrucksignals langfristig liegen sollte. Eine Abflussstenose mit einer Amplitude des Herzdrucksignals von > 20 mmHg kann den Fistelfluss bereits deutlich einschränken. Ein solcher Bereich kann allgemeingültig sein oder für jeden Patienten neu ermittelt werden. Wie in Fig. 3 gezeigt, können mit Hilfe einer Langzeitüberwachung der Amplitude des Herzdrucksignals entsprechend Veränderungen am Gefäßzugang erkannt werden.

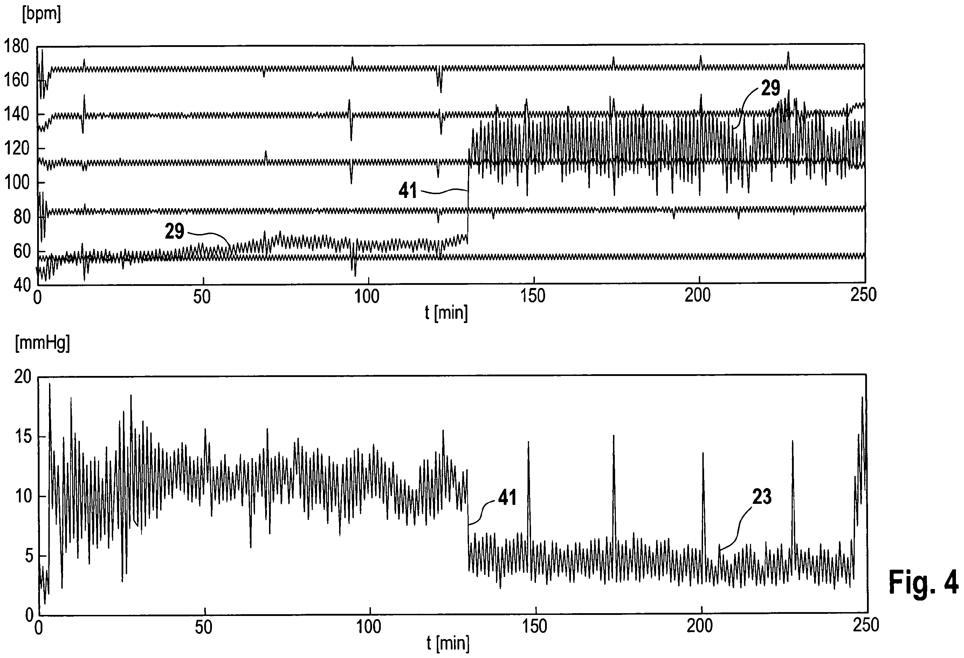

Fig. 4 zeigt Graphen einer Herzfrequenz 29 (oben) und einer Amplitude eines Herzdrucksignals 23 (unten) über der Zeit. Der in beiden Graphen nach annähernd 130 Minuten auftretende Sprung zum Zeitpunkt 41 spiegelt einen Übergang in intermittierendes Vorhofflimmern wieder. Ein solcher Vorgang führt zu hohem unregelmäßigen Puls, wie in Fig. 4 oben zu erkennen ist.

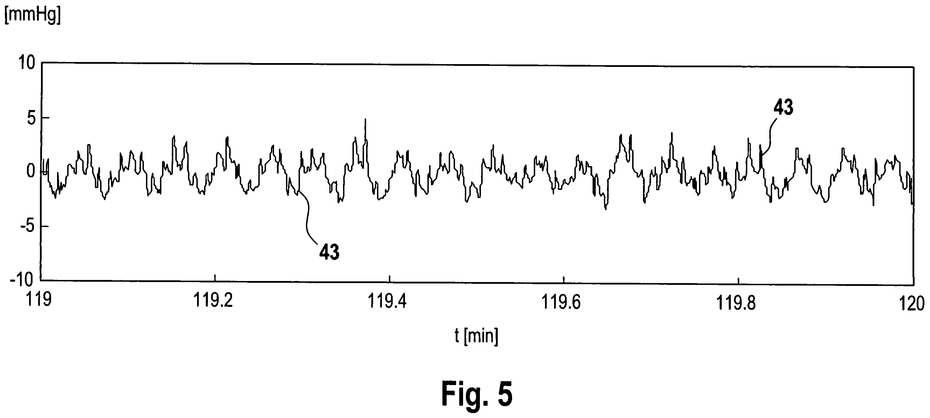

Fig. 5 stellt grafisch eine Überlagerung 43 von Einflüssen von Atmung und Herzdrucksignal über der Zeit dar. Die Überlagerung 43 setzt sich aus kleinen Peaks des Herzschlags und großen Schwankungen der Atmung zusammen. Die Atmung ist insbesondere bei katheterisierten Patienten bei Messung im rechten Vorhof darstellbar. Der intrathorakale Druck der Atmung ist wie in Fig. 5 gezeigt erfassbar.

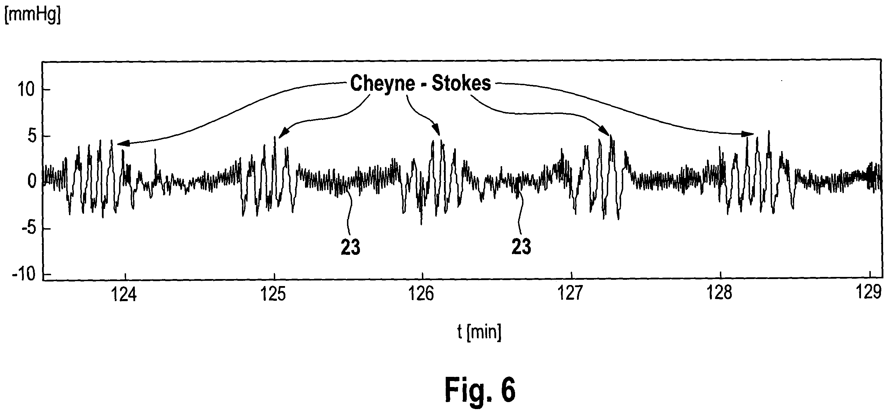

Fig. 6 zeigt eine Cheyne-Stokes-Atmung. Bei dieser erfolgen fünf bis sechs aufeinander folgende Atemzüge, gefolgt von einer Atempause. In der Atempause ist anhand der charakteristischen Amplitude des Herzdrucksignals 23 die Herzpulsation gut erkennbar.

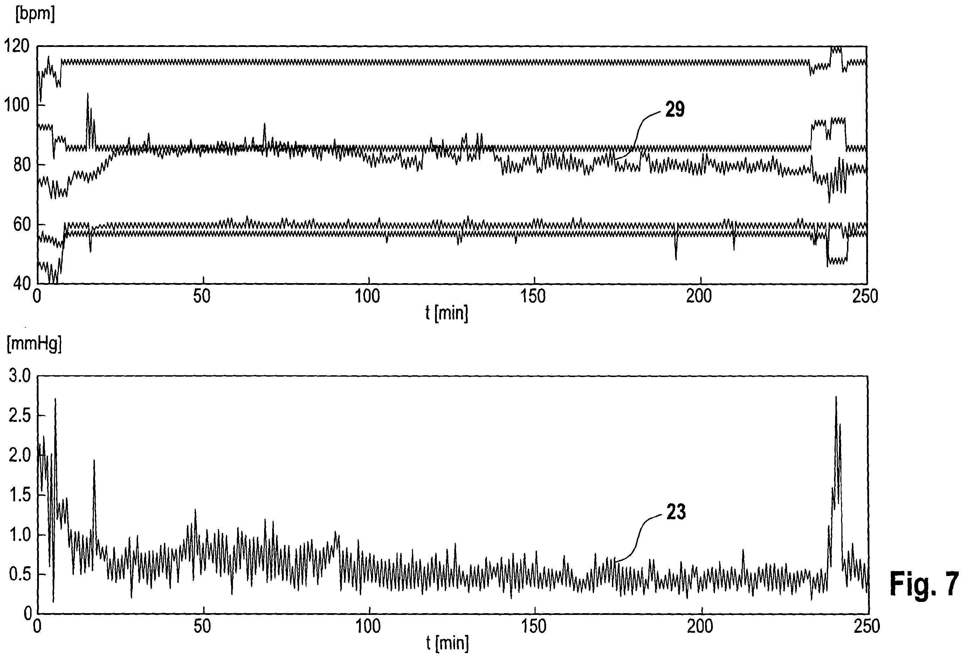

Fig. 7 zeigt in einem weiteren Graphen eine Herzfrequenz (oben) und die Amplitude eines Herzdrucksignals (unten) über der Zeit. Die entsprechenden Daten wurden von einem Herzschrittmacher-Patienten erhalten. Der Herzschrittmacher "greift" nur gelegentlich ein, was zu den zwei unterschiedlichen, dargestellten Herzraten führt.

Fig. 8 zeigt schematisch die Extraktion eines Herzdrucksignals 23 aus einem Messsignal 45, welches neben dem Herzdrucksignal auch ein Pumpensignal aufweist (oben), und ein entsprechendes Histogramm (unten). Die Extraktion des Herzdrucksignals 23 erfolgt durch Erfassen von Hallsensorsignalen einer Blutpumpe, wie beispielsweise einer Blutpumpe einer Dialysemaschine aus der Maschinengeneration mit der Bezeichnung 5008 aus dem Hause Fresenius Medical Care. Dabei geben die Kreise 47 die hallsensorsynchrone Abtastung wieder. Die Kreise 49 geben das Herzdrucksignal zum Zeitpunkt der Hallsensorimpulse an. Abgetastet wird bei jedem Hallsensorimpuls. Das Histogramm gibt die Werte der Kreise 49 wieder. Standardabweichungen können hierbei als Maß für die Stärke der Pulsation verstanden werden. Der Mittelwert im Histogramm kann eine Aussage über den Fisteldruck geben bzw. ermöglichen. Als Maß für die Herzdruckamplitude kann beispielsweise das Mittel +/- einer Standardabweichung dienen. Möglich ist auch eine Verwendung eines Perzentils (10., 90., usw.), eines Perzentilbereichs oder Kombinationen hieraus.

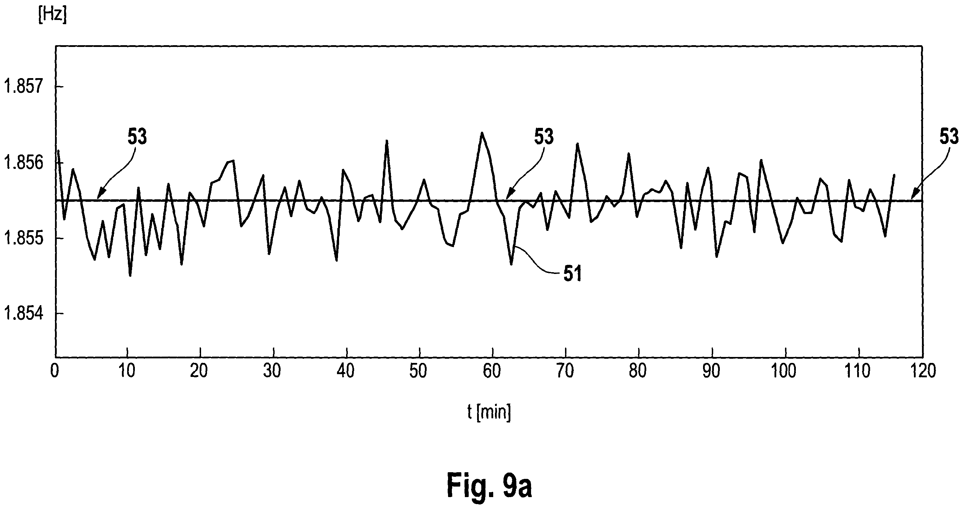

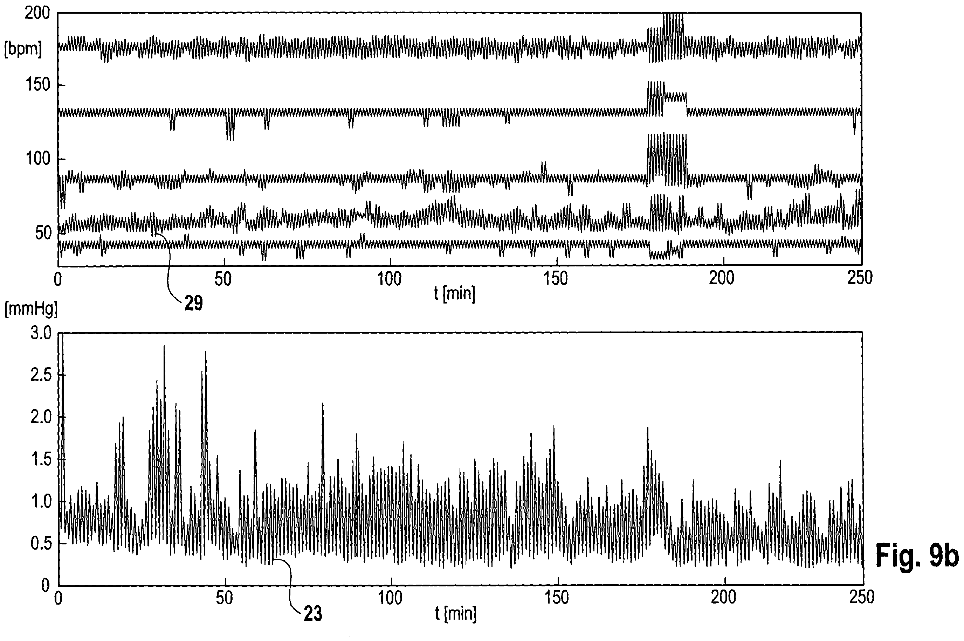

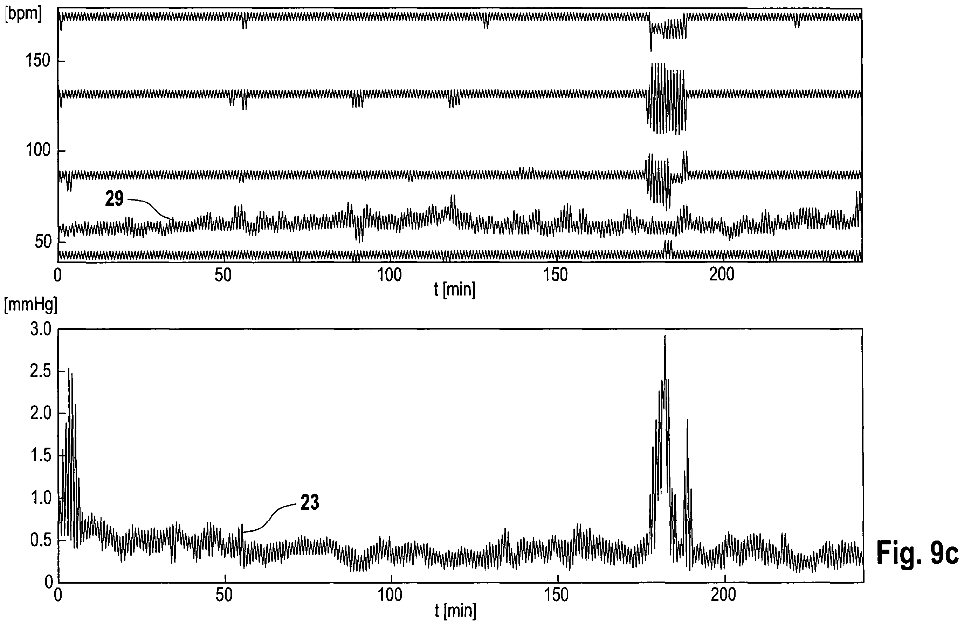

Fig. 9 zeigt schematisch den Unterschied zwischen einer zeitsynchronen (Fig. 9b) und einer winkelsynchronen (Fig. 9c) Abtastung des Pumpensignals. Anders als in Fig. 8 gezeigt, kann das arterielle Drucksignal auch winkelsynchron zu einem Pumpenrotor abgetastet werden. Dies kann die Extraktion des Herzdrucksignals verbessern, vor allem wenn die Blutpumpe nicht vollkommen gleichmäßig rotiert, d. h wenn die Pumpenfrequenz 51 von einer Sollfrequenz 53 abweicht wie in Fig. 9a gezeigt. Dabei steht die Einheit [Hz] für die Pumpenfrequenz.

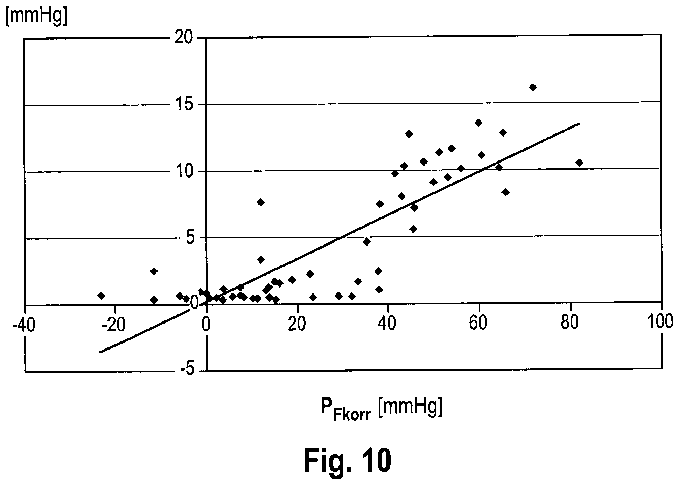

Fig. 10 zeigt einen Graphen eines Herzdrucksignals 23 über einem hydrostatisch korrigierten Fisteldruck P_{Fkorr}(an der arteriellen Nadel innerhalb der Fistel) mit Werten aus mehr als 50 Dialysebehandlungen. Es besteht ein Zusammenhang zwischen dem Fisteldruck und der Amplitude des Herzdrucksignals. Dies liegt zum einen am Rückgang des Schlagvolumens: Bei sinkendem Blutangebot während einer Behandlung sinkt auch das Schlagvolumen, was kompensatorisch zu erhöhter Herzrate führt. Zum anderen ist dies durch eine Abnahme des Fisteldrucks erklärbar. Es kann auf eine relative Änderung des Fisteldrucks geschlossen werden, wenn der Effekt des Schlagvolumens zum Beispiel über die Herzrate korrigiert wird. Der Zusammenhang zwischen Fisteldruck und Amplitude kann auch in der Elastizität der Fistel begründet liegen. Eine prall gefüllte Fistel kann sich nicht weiter dehnen und überträgt Herzpulse mit geringerer Dämpfung oder nahezu ohne Dämpfung. Eine leere, schlaffe Fistel dämpft die Pulsation stärker. Ferner kann durch Bestimmen der Amplitude bei verschiedenen Drehzahlen der Blutpumpe auf das Blutangebot der Fistel geschlossen und somit eine Schätzung des Fistelflusses vorgenommen werden.

## Patentansprüche

1. Vorrichtung zum Durchführen eines Verfahrens zum Ermitteln und/oder Überwachen wenigstens eines körperlichen Zustandes eines Patienten (1) mit einer Einrichtung, welche konfiguriert ist zum Ermitteln einer Amplitude eines Drucksignals (21) während einer mittels einer Blutbehandlungsvorrichtung (5), welche eine Blutpumpe (13) aufweist und mit einem extrakorporalen Blutkreislauf verbunden ist, durchgeführten Blutbehandlung des Patienten (1), wobei das Drucksignal (21) ein Herzdrucksignal (23) umfasst, und wobei die Einrichtung konfiguriert ist, das Drucksignal (21) mittels eines Drucksensors (11) auf der arteriellen Seite des extrakorporalen Blutkreislaufs zu ermitteln;
einer Einrichtung, welche konfiguriert ist zum Korrigieren des ermittelten Wertes der Amplitude des Drucksignals (21) um einen Beitrag (25) der Blutbehandlungsvorrichtung zur Höhe der Amplitude des Drucksignals (21) und zum Ermitteln einer Amplitude des Herzdrucksignals (23) aus dem Drucksignal (21); und einer Einrichtung, welche konfiguriert ist zum Auswerten der Amplitude des ermittelten Drucksignals (21) über eine Mehrzahl von Dialysebehandlungen zum Treffen einer Aussage über den körperliche Zustand, wobei der Zustand eine sich entwickelnde Abflussstenose einer Fistel ist, wobei die Einrichtung dazu konfiguriert ist, die über einen Beobachtungszeitraum, welcher die Mehrzahl von Dialysebehandlungen umspannt, ermittelte Amplitude des Herzdrucksignals (23) mit einem definierten Bereich zu vergleichen, wobei der Bereich ein Bereich ist, in dem die Amplitude des Herzdrucksignals langfristig liegen sollte.

2. Vorrichtung nach Anspruch 1, wobei es sich bei der Blutbehandlungsvorrichtung (5) um eine Vorrichtung zur Hämodialyse, Hämofiltration oder Hämodiafiltration handelt.

3. Vorrichtung nach Anspruch 1 oder 2, wobei die Einrichtung zum Korrigieren des ermittelten Wertes der Amplitude des Drucksignals (21) um einen Beitrag (25) der Blutbehandlungsvorrichtung konfiguriert ist zum Korrigieren des ermittelten Wertes der Amplitude des Drucksignals (21) in Abhängigkeit von einem Positionssensor oder einem als Hallsensor ausgestalteten Positionssensor übermittelten Signal.

4. Vorrichtung nach einem der Ansprüche 1 bis 3, wobei die Einrichtung zum Korrigieren des ermittelten Wertes der Amplitude des Drucksignals (21) um einen Beitrag (25) der Blutbehandlungsvorrichtung konfiguriert ist zum Korrigieren des ermittelten Wertes der Amplitude des Drucksignals (21) in Abhängigkeit von einem Drehwinkel der Blutpumpe (13) der Blutbehandlungsvorrichtung (5), wobei die Blutpumpe (5) eine peristaltische Blutpumpe ist.

5. Vorrichtung nach einem der Ansprüche 1 bis 4, mit einer Ausgabeeinrichtung zum Ausgeben eines Ergebnisses der Auswertung.

6. Blutbehandlungsvorrichtung, welche eine Vorrichtung nach einem der Ansprüche 1 bis 5 aufweist, und welche die Blutpumpe (13), einen Dialysator (15), einen Drucksensor (17) auf der venösen Seite und eine Tropfkammer (19) aufweist.

## Claims

1. Apparatus for carrying out a method to determine and/or to monitor at least one physical state of a patient (1) comprising:
- a device configured to determine an amplitude of a pressure signal (21) during a blood treatment of the patient (1) executed by means of a blood treatment apparatus (5) comprising a blood pump (13) and being connected with an extracorporeal blood circuit, wherein the pressure signal (21) comprises a heart pressure signal (23) and wherein the device is configured to determine the pressure signal (21) by means of a pressure sensor (11) on the arterial side of the extracorporeal blood circuit;
- a device configured to correct the determined value of the amplitude of the pressure signal (21) by a contribution (25) of the blood treatment apparatus to the height of the amplitude of the pressure signal (21) and to determine an amplitude of the heart pressure signal (23) from the pressure signal (21); and
- a device configured to evaluate the amplitude of the determined pressure signal (21) over a plurality of dialysis treatments for drawing a conclusion about the physical state, wherein the state is the development of an outflow stenose from a fistula, wherein the device is configured to compare the determined amplitude of the heart pressure signal (23) with a defined range during an observation period comprising a plurality of dialysis treatments, wherein the range is a range within which the amplitude of the heart pressure signal should lie in a longer term.

2. The apparatus according to claim 1, wherein the blood treatment apparatus (5) is an apparatus for the hemodialysis, hemofiltration or hemodiafiltration.

3. The apparatus according to claim 1 or 2, wherein the device for correcting the determined value of the amplitude of the pressure signal (21) of the contribution (25) of the blood treatment apparatus is configured to correct the determined value of the amplitude of the pressure signal (21) depending on a transmitted signal of a position sensor or of a position sensor designed as a Hall sensor.

4. The apparatus according to anyone of claims 1 to 3, wherein the device for correcting the determined value of the amplitude of the pressure signal (21) of the contribution (25) of the blood treatment apparatus is configured to correct the determined value of the amplitude of the pressure signal (21) depending on a rotation angle of the blood pump (13) of the blood treatment apparatus (5), wherein the blood pump (13) is a peristaltic blood pump.

5. The apparatus according to anyone of claims 1 to 4 comprising an output device for outputting a result of the evaluation.

6. Blood treatment apparatus comprising an apparatus according to anyone of claims 1 to 5 and comprising the blood pump (13), a dialyser (15), a pressure sensor (17) on the venous side and a drip chamber (19).

## Revendications

1. Appareil pour la mise en oeuvre d'un procédé permettant de déterminer et/ou de surveiller au moins un état physique d'un patient (1) comprenant:
- un dispositif configuré pour déterminer une amplitude d'un signal de pression (21) au cours d'un traitement du sang du patient (1) effectué au moyen d'un appareil de traitement du sang (5) comprenant une pompe à sang (13) et étant relié à un circuit sanguin extracorporel, où le signal de pression (21) comprend un signal de pression cardiaque (23) et où le dispositif est configuré de façon à déterminer le signal de pression (21) au moyen d'un capteur de pression (11) du côté artériel du circuit sanguin extracorporel;
- un dispositif configuré pour corriger la valeur déterminée de l'amplitude du signal de pression (21) d'une contribution (25) de l'appareil de traitement du sang à la hauteur de l'amplitude du signal de pression (21) et pour déterminer une amplitude du signal de pression cardiaque (23) à partir du signal de pression (21); et
- un dispositif configuré pour évaluer l'amplitude du signal de pression (21) déterminé sur plusieurs traitements de dialyse afin de conclure l'état physique, où l'état physique est le développement d'une sténose de décharge d'une fistule, où le dispositif est configuré de façon à comparer l'amplitude déterminée du signal de pression cardiaque (23) avec un domaine de valeurs défini durant une période d'observation s'étendant sur plusieurs traitements de dialyse, où le domaine de valeurs est un domaine dans lequel l'amplitude du signal de pression cardiaque devrait se situer à long terme.

2. L'appareil selon la première revendication, où ledit appareil de traitement du sang (5) est un appareil d'hémodialyse, d'hémofiltration ou d'hémodiafiltration.

3. L'appareil selon la revendication 1 ou 2, où le dispositif pour corriger la valeur déterminée de l'amplitude du signal de pression (21) d'une contribution (25) de l'appareil de traitement du sang est configuré de façon à corriger la valeur déterminée de l'amplitude du signal de pression (21) en fonction d'un signal transmis d'un capteur de position ou d'un capteur de position conçu comme un capteur à effet Hall.

4. L'appareil selon les revendications 1 à 3, où le dispositif pour corriger la valeur déterminée de l'amplitude du signal de pression (21) d'une contribution (25) de l'appareil de traitement du sang est configuré de façon à corriger la valeur déterminée de l'amplitude du signal de pression (21) en fonction d'un angle de rotation de la pompe à sang (13) de l'appareil de traitement du sang (5), où la pompe à sang (13) est une pompe à sang péristaltique.

5. L'appareil selon l'une quelconque des revendications 1 à 4, comprenant un dispositif de sortie pour émettre un résultat de l'évaluation.

6. L'appareil de traitement du sang comprenant un appareil selon l'une quelconque des revendications 1 à 5 et comprenant la pompe à sang (13), un dialyseur (15), un capteur de pression (17) du côté veineux ainsi qu'une chambre compte-gouttes (19).
